# EUROPEAN PATENT SPECIFICATION

(11) **EP 2 301 426 B1**
(45) Date of publication and mention of the grant of the patent: **17.02.2016**
(21) Application number: 10178552.5
(22) Date of filing: 23.09.2010
(51) Int. Cl.: A61B 5/00, A61B 5/11, G08B 21/22

(54) **Biological information monitor having function of displaying temporary room leaving timer**
Biologischer Informationsmonitor mit Funktion zur Anzeige einer Zeituhr zum zeitweisen Verlassen des Raums
Moniteur d'informations biologiques disposant d'une fonction d'affichage à temporisateur temporaire

(30) Priority: 24.09.2009 JP 2009219492
(43) Date of publication of application: 30.03.2011
(73) Proprietor: Nihon Kohden Corporation, Shinjuku-ku Tokyo (JP)
(72) Inventor: Tezuka, Shinji, Shinjuku-ku Tokyo (JP)
(74) Representative: Grünecker Patent- und Rechtsanwälte PartG mbB

(56) References cited:
- EP-A2- 1 238 626
- EP-A2- 1 363 257
- JP-A- 2002 143 101
- US-A1- 2007 040 692
- US-A1- 2007 156 031
- US-A1- 2007 194 939

## Description

### BACKGROUND OF THE INVENTION

The present invention relates to a biological information monitor that includes a display portion in which biological information obtained by processing a biological signal detected from a living body is displayed.

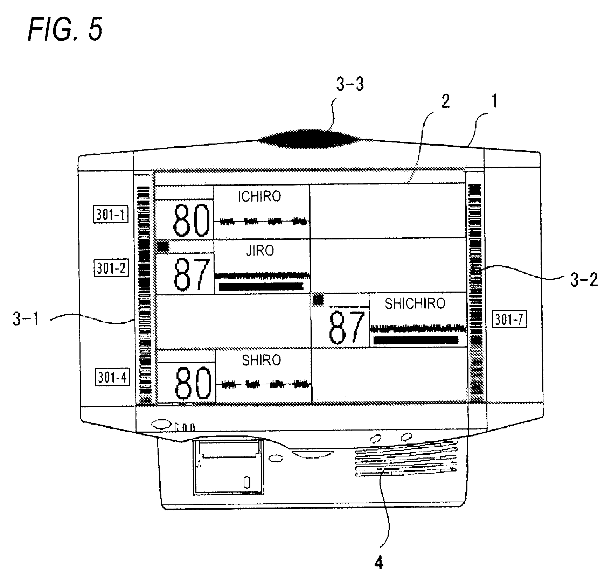

Fig. 5 shows an example of the whole configuration of a biological information monitor.

Fig. 5 is an external view of a biological information monitor of the central monitor type which is placed in a nurse's station or the like, and in which biological information of a plurality of patients can be simultaneously monitored.

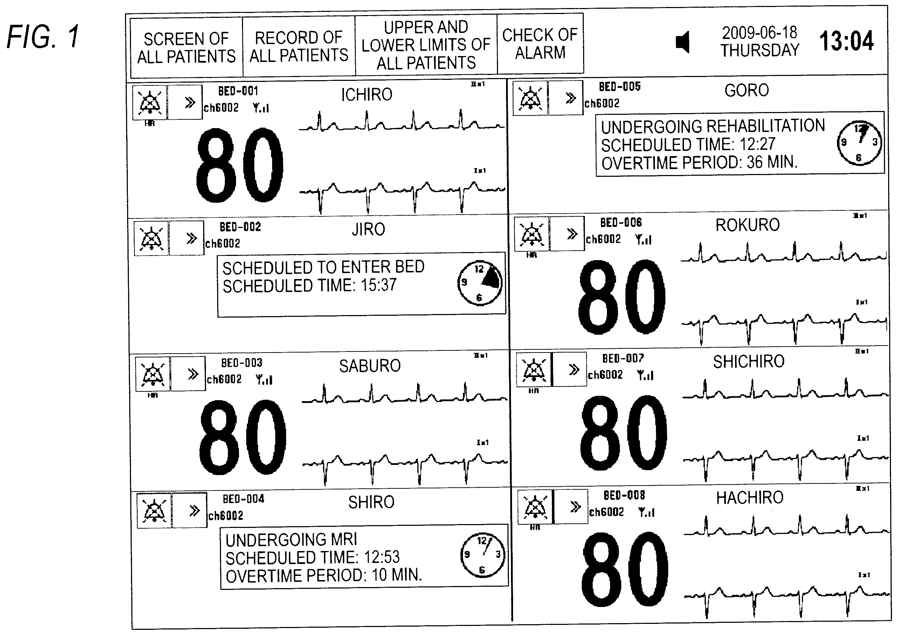

In Fig. 5, 1 denotes the biological information monitor of the central monitor type, and 2 denotes a display screen on which numerals and waveforms that are obtained as a result of processing biological information of a plurality of patients are simultaneously displayed.

The reference numerals 3-1, 3-2, and 3-3 denote display output portions for a visual alarm. The display output portions 3-1, 3-2 are individual display portions which can perform displays respectively corresponding to individual patients, and the display output portion 3-3 is a common display portion which is common to all patients. Each of the display output portions 3-1, 3-2, and 3-3 is configured by, for example, LEDs, and selectively displays red, yellow, or cyan light, or like alarm light in accordance with the priority of the alarm.

The reference numeral 4 is a speaker which outputs an audible alarm. The speaker 4 can output an alarm corresponding to the priority by means of different frequencies, duration times, and the like.

In the biological information monitor of Fig. 5, as illustrated, biological information of eight patients can be simultaneously displayed on the display screen. In the illustrated state, however, biological information of only four patients (ICHIRO, JIRO, SHIRO, and SHICHIRO) is displayed.

In a nursing home, a hospital, or the like, a nurse or a helper patrols rooms to check whether elderly persons or hospital patients are present in respective beds disposed in the rooms or not. By means of such a patrol, the nursing home or the hospital know sleeping, wandering, and the like of hospital patients, however it is difficult to monitor all of elderly persons or hospital patients. In order to solve this problem, there is the following art disclosed in JP-A-2005-185650.

JP-A-2005-185650 discloses a system which determines the absence of an elderly persons, a hospital patient, or the like, and which notifies a nurse's station or the like of a result of the determination. Specifically, the system disclosed in JP-A-2005-185650 detects the value of a load applied to a bed, and determines the presence or absence of a person in the bed, depending on the load. In such a system in which a load due to the human body is detected to determine the presence or absence of the user in a bed, a strain resistor or load cell which detects the weight of the human body, or a sensor which utilizes deformation of a coil spring or the like is used. Further, in the system, when the load value is equal to or larger than a predetermined value and continues for a fixed time period or longer, the measurement for absence from bed detection is started. In the case where, after the start of the measurement, the load value is equal to or smaller than a predetermined value and continues for a fixed time period or longer, the system determines that absence from a bed occurs.

In the biological information monitor of Fig. 5, as illustrated, biological information of eight patients can be simultaneously displayed on the display screen. In the illustrated state, however, biological information of only four patients (ICHIRO, JIRO, SHIRO, and SHICHIRO) is displayed.

Since biological information of the remaining four patients is not displayed on the display screen, however, a medical person who monitors the states of the patients, in a nurse's station or the like where the biological information monitor is placed cannot know whether the remaining four patients temporarily leave respective beds because of a reason such as inspection (this state is referred to as temporary room leaving), or the biological information cannot be displayed because of drop off of measuring means for measuring biological information from the patients or failures of measuring apparatuses. Consequently, there may arise a problem in monitoring of biological information of the patients.

Even in the case where it is known that the patients are in temporary room leaving because of a reason such as inspection, it is impossible to know when the patients return to the beds, and hence there is a possibility that the medical person forgets to restart the measurement. US 2007/0194939 A1 provides a patient information and communication method and a system for a healthcare facility in which an user interface in provided in a in-patient hospital room and workstation in communication with the in-patient hospital room. A display portion of the workstation displays biological information of all patients and, in addition, a multiple click buttons are provided to view further information such as the scheduled task for each patient, current status of the patient and the likes. When current status of all the patients is to be known, however, this multiple click could be a burden.

If the medical person can previously know the times when the patients return to the beds, the medical person can perform a work in preparation for returning.

### SUMMARY

It is therefore an object of the invention to provide a biological information monitor having a function of displaying a temporary room leaving timer which, when a patient temporarily leaves the room because of a reason such as inspection, displays the reason of the temporary room leaving accompanied by stoppage of measurement of biological information of the patient, the period for leaving the room, the scheduled time for restarting the measurement, and present progress information, in a display area for the patient, thereby preventing restart of measurement of biological information when the patient returns to the room (enters the bed), from being forgotten.

In order to achieve the object, according to the invention, there is provided a biological information monitor as defined in claim 1.

The further embodiments are defined in dependent claims.

### BRIEF DESCRIPTION OF THE DRAWINGS

Fig. 1 is a view showing an example of a mode of, in the case where a patient temporarily leaves the room because of a reason such as inspection, displaying the reason of the temporary room leaving accompanied by stoppage of measurement of biological information of the patient, the time of returning to the room (scheduled time for restarting the measurement), and present progress information, in the biological information monitor having a function of displaying a temporary room leaving timer of the invention.
Fig. 2 is a view showing an input screen for inputting the reason for temporary room leaving, the period for leaving the room, and the scheduled time for restarting the measurement.
Figs. 3A to 3C are views showing a display state in the case where, after a setting process is performed through the input screen, a time period has elapsed.
Figs. 4A to 4D are views showing a display mode other than an analog clock display shown in Figs. 3A to 3C.
Fig. 5 is an external view of a biological information monitor of the central monitor type which is placed in a nurse's station or the like, and in which biological information of a plurality of patients can be simultaneously monitored.

### DETAILED DESCRIPTION OF EMBODIMENTS

The configuration of the invention will be described with reference to Fig. 1.

Referring to Fig. 1, a mode of displaying the reason of the temporary room leaving accompanied by stoppage of measurement of biological information of the patient, the period for leaving the room, the scheduled time for restarting the measurement, and present progress information will be described.

In the biological information monitor of Fig. 1, a display of the present time of 13:04, and biological information of eight patients (ICHIRO, JIRO, SABURO, SHIRO, GORO, ROKURO, SHICHIRO, and HACHIRO) can be simultaneously displayed. As biological information, however, biological information of only five patients (ICHIRO, SABURO, ROKURO, SHICHIRO, and HACHIRO) is displayed.

With respect to a patient (JIRO), the patient has not yet hospitalized (entered the bed), and hence 15:37 is displayed as the scheduled time for entering the bed, in the display area for biological information.

With respect to another patient (SHIRO), displays of "UNDERGOING MRI" as the reason of the temporary room leaving, 12:53 as the scheduled time, and the overtime period of 10 min. are displayed. The overtime period is displayed in the form of an analog clock.

With respect to a further patient (GORO), displays of "UNDERGOING REHABILITATION" as the reason of the temporary room leaving, 12:27 as the scheduled time, and the overtime period of 36 min. are displayed. The overtime period is displayed in the form of an analog clock.

The biological information monitor of the invention includes a visual alarm and an audible alarm which are similar to visual alarms 3-1, 3-2, 3-3 and an audible alarm 4 of the biological information monitor shown in Fig. 5. In the case where the overtime period is displayed (the patient does not return until the scheduled time), for example, an output may be performed through one of the visual alarm and the audible alarm.

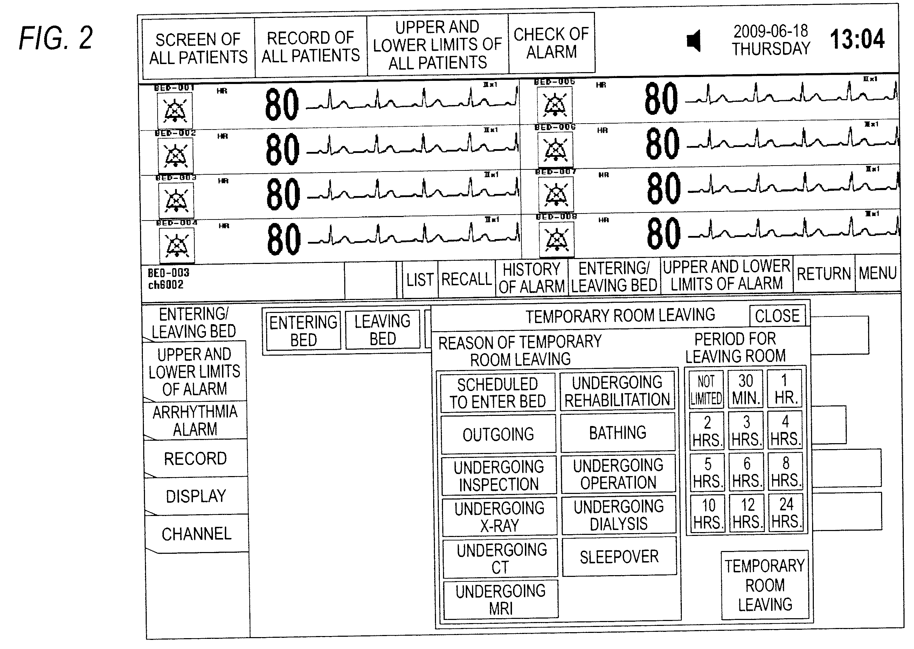

Next, means for inputting the reason for temporary room leaving, the period for leaving the room, and the scheduled time for restarting the measurement in the biological information monitor of the invention will be described with reference to Fig. 2.

Fig. 2 shows a state where, in the case where a function of "ENTERING/LEAVING BED" not shown in Fig. 1 is selected in the state in which the display screen of Fig. 1 is displayed, the display screen shown in Fig. 1 is compressed to an upper part of a display portion, and an input screen for the reason for temporary room leaving, the period for leaving the room, and the scheduled time for restarting the measurement is displayed.

In the input screen, selection items of "SCHEDULED TO ENTER BED", "UNDERGOING REHABILITATION", "OUTGOING", "BATHING", "UNDERGOING INSPECTION" "UNDERGOING OPERATION", "UNDERGOING X-RAY", "UNDERGOING DIALYSIS", "UNDERGOING CT", "SLEEPOVER", and "UNDERGOING MRI" are disposed as reasons of the temporary room leaving.

It is obvious that, in addition to these items, other items may be set as the reason of the temporary room leaving.

As the period for leaving the room, selection items of "NOT LIMITED", "30 MIN." "1 HR.", "2 HRS.", "3 HRS.", "4 HRS.", "5 HRS.", 6 HRS.", "8 HRS.", "10 HRS.", "12 HRS.", and "24 HRS." are disposed.

It is obvious that, in addition to these items, other items may be set as the period for leaving the room.

In the input screen, when the reason of the temporary room leaving and the period for leaving the room are selected and a button of "TEMPORARY ROOM LEAVING" is then pressed, the reason for temporary room leaving accompanied by stoppage of measurement of biological information of the patient, the period for leaving the room, the scheduled time for restarting the measurement, and the present progress information are displayed by a controlling portion (CPU) (not shown) in view of the present time in the form of an analog clock.

After the setting process is performed through the input screen of Fig. 2, the set time period may be set to be shortened or prolonged.

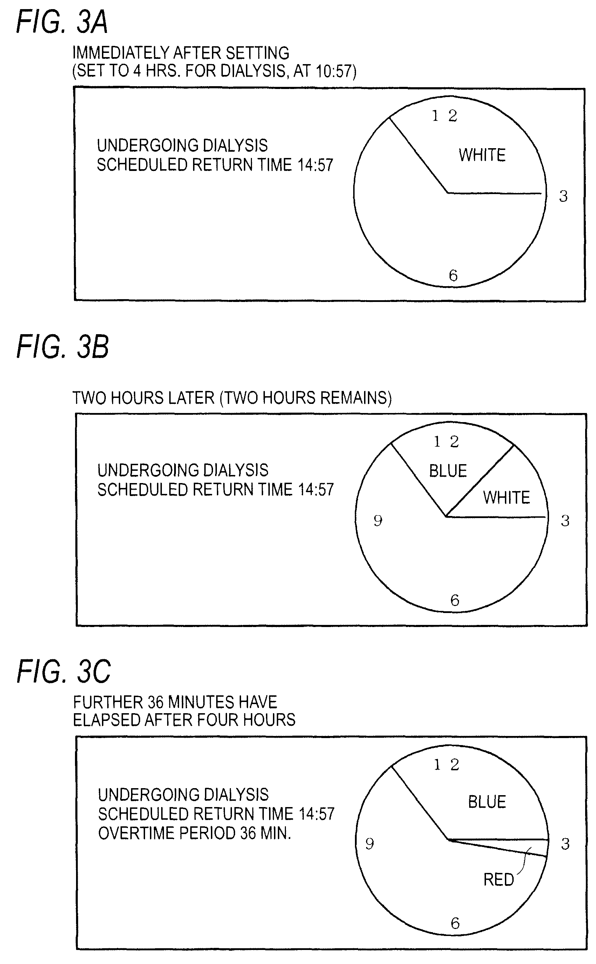

Next, a display state in the case where, after a setting process is performed through the input screen of Fig. 2, a time period has elapsed will be described with reference to Figs. 3A to 3C.

Fig. 3A shows a display state which is obtained immediately after, in the input screen of Fig. 2, "UNDERGOING DIALYSIS" is selected as the reason of the temporary room leaving, "4 HRS. " is selected as the period for leaving the room, and the button of "TEMPORARY ROOM LEAVING" is pressed at 10: 57.

In the display area for the patient, characters of "UNDERGOING DIALYSIS" and "SCHEDULED RETURN TIME 14:57" are displayed, and, in the form of an analog clock, the region between 10: 57 and 14 : 57 is displayed by "white" as the remaining time period of dialysis.

Fig. 3B shows a display state which is obtained after two hours have elapsed from the time when, in the input screen of Fig. 2, "UNDERGOING DIALYSIS" is selected as the reason of the temporary room leaving, "4 HRS." is selected as the period for leaving the room, and the button of "TEMPORARY ROOM LEAVING" is pressed at 10:57.

In the display area for the patient, characters of "UNDERGOING DIALYSIS" and "SCHEDULED RETURN TIME 14:57" are displayed, and, in the form of an analog clock, the region between 10:57 and 12:57 is displayed by "blue" as the elapsed time period, and the region between 12: 57 and 14 : 57 is displayed by "white" as the remaining time period of dialysis.

Fig. 3C shows a display state which is obtained after four hours and 36 minutes have elapsed from the time when, in the input screen of Fig. 2, "UNDERGOING DIALYSIS" is selected as the reason of the temporary room leaving, "4 HRS." is selected as the period for leaving the room, and the button of "TEMPORARY ROOM LEAVING" is pressed at 10:57.

In the display area for the patient, characters of "UNDERGOING DIALYSIS", "SCHEDULED RETURN TIME 14:57" and "OVERTIME PERIOD 36 MIN." are displayed, and, in the form of an analog clock, the region between 10: 57 and 14 : 57 is displayed by "blue" as the elapsed time period, and the region between 14:57 and 15:33 is displayed by "red" as the overtime period.

In the case where an overtime period is displayed (the patient does not return until the scheduled time), for example, an output may be performed through one of the visual alarm and the audible alarm.

As described above, according to the biological information monitor of the invention, biological information which is measured by a biological information detection portion attached to the patient is displayed directly or after processed, in a biological information display area of the display portion, and, in the display area, the reason of the temporary room leaving accompanied by stoppage of measurement of biological information of the patient, the period for leaving the room and the scheduled time for restarting the measurement (the time of returning to the room) are displayed in a form in which the reason and the time are easily recognized by a medical person. When the patient returns to the room (enters the bed), therefore, it is possible to prevent restart of measurement of biological information from being forgotten.

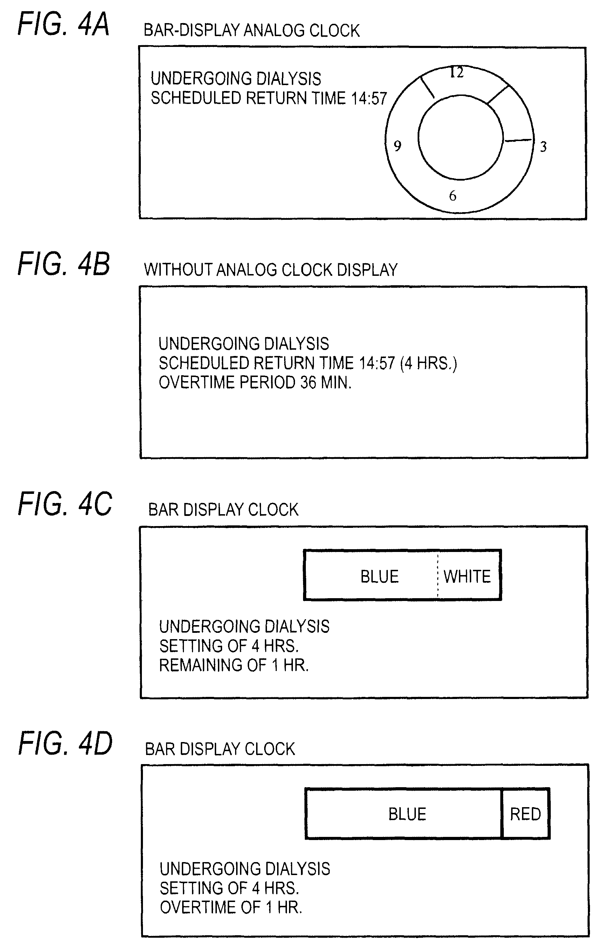

As the displaying mode, in place of the analog clock display mode shown in Figs. 3A to 3C, a bar display analog clock shown in Fig. 4A may be employed.

Fig. 4A shows a state which is identical with the display state of Fig. 3B which is obtained after two hours have elapsed from the time when, in the input screen of Fig. 2, "UNDERGOING DIALYSIS" is selected as the reason of the temporary room leaving, "4 HRS." is selected as the period for leaving the room, and the button of "TEMPORARY ROOM LEAVING" is pressed at 10:57.

In the display area for the patient, characters of "UNDERGOING DIALYSIS" and "SCHEDULED RETURN TIME 14:57" are displayed, and, in the form of a bar display analog clock, the region between 10:57 and 12:57 is displayed by "blue" as the elapsed time period, and the region between 12:57 and 14:57 is displayed by "white" as the remaining time period of dialysis.

The bar display may be expressed in a linear form as shown in Figs. 4C and 4D, in place of the annular form in the analog clock of Fig. 4A.

The displaying mode may be performed only by a character display as shown in Fig. 4B, in place of the analog clock display mode shown in Figs. 3A to 3C.

According to an aspect of the invention, when a patient temporarily leaves the room because of a reason such as inspection, the reason of the temporary room leaving accompanied by stoppage of measurement of biological information of the patient, the period for leaving the room, the scheduled time for restarting the measurement, and present progress information are displayed in the display area for the patient, thereby enabling the time when the patient is to return to the room (enter the bed), to be easily known simply by viewing the display screen for the patient. Therefore, it is possible to prevent restart of measurement of biological information from being forgotten, and the workability of a medical person can be improved.

## Claims

1. A biological information monitor adapted to receive, directly or via network, biological information of a patient which is measured by a unit attached to the patient, the biological information monitor comprising:
a display portion on which the biological information is displayed;
an inputting portion for inputting a reason for temporarily leaving a room, and a period for temporarily leaving the room, and for starting the period of temporarily leaving the room; and
a CPU configured to simultaneously display, on the display portion, the reason for temporarily leaving the room accompanied by stoppage of measurement of the biological information of the patient, the period for leaving the room, and, in view of a present time, a scheduled time for re-entering bed for restarting the measurement, and present progress information of time including information of overtime related to the scheduled time.

2. The biological information monitor according to claim 1, wherein
the inputting portion includes a plurality of items for the reason and a plurality of items for the period, and
the reason is selected from the items for the reason and the period is selected from the items for the period.

3. The biological information monitor according to claim 1, further comprising: an alarm outputting portion configured to output an alarm including a visual or audible alarm, wherein
in a case where the measurement is stopped after the scheduled time for restarting the measurement, the alarm is output.

4. The biological information monitor according claim 1, wherein the CPU is further configured to graphically display the present progress information and graphically distinguishing the present time, an elapsed time, a remaining time period, and an overtime period from one another.

5. The biological information monitor according claim 4, wherein the CPU is further configured to display the present progress information in a form of an analog clock.

6. The biological information monitor according claim 4, wherein the CPU is further configured to display the present progress information in a form of a bar graph display.

7. The biological information monitor according to claim 1, wherein, in the inputting portion, an input comment is input together with the reason and the period.

8. The biological information monitor according to claim 1, being a monitor in which biological information of a plurality of patients is displayed on a common display portion and remotely monitored.

## Patentansprüche

1. Vorrichtung zum Überwachen biologischer Informationen, die so eingerichtet ist, dass sie direkt oder über ein Netzwerk biologische Informationen eines Patienten empfängt, die mit einer an dem Patienten angebrachten Einheit gemessen werden, wobei die Vorrichtung zum Überwachen biologischer Informationen umfasst:
einen Anzeigeabschnitt, an dem die biologischen Informationen angezeigt werden;
einen Eingabeabschnitt, über den ein Grund zum vorübergehenden Verlassen eines Raums sowie ein Zeitraum zum vorübergehenden Verlassen des Raums eingegeben werden und der Zeitraum zum vorübergehenden Verlassen des Raums gestartet wird; sowie
eine CPU, die so eingerichtet ist, dass sie auf dem Anzeigeabschnitt gleichzeitig den Grund zum vorübergehenden Verlassen des Raums zusammen mit Unterbrechung von Messung der biologischen Informationen des Patienten, den Zeitraum zum Verlassen des Raums und, unter Berücksichtigung der aktuellen Zeit, eine geplante Zeit zum Zurückkehren zum Bett und zum erneuten Starten der Messung sowie aktuelle Fortschritts-Informationen bezüglich der Zeit einschließlich Informationen über Überziehung der geplanten Zeit anzeigt.

2. Vorrichtung zum Überwachen biologischer Informationen nach Anspruch 1, wobei
der Eingabeabschnitt eine Vielzahl von Elementen für den Grund sowie eine Vielzahl von Elementen für den Zeitraum enthält, und
der Grund aus den Elementen für den Grund ausgewählt wird und der Zeitraum aus den Elementen für den Zeitraum ausgewählt wird.

3. Vorrichtung zum Überwachen biologischer Informationen nach Anspruch 1, die des Weiteren einen Warnungs-Ausgabeabschnitt umfasst, der so eingerichtet ist, dass er eine Warnung ausgibt, die eine optische oder eine akustische Warnung einschließt, wobei
wenn die Messung nach der geplanten Zeit zum erneuten Starten der Messung unterbrochen wird, die Warnung ausgegeben wird.

4. Vorrichtung zum Überwachen biologischer Informationen nach Anspruch 1, wobei die CPU des Weiteren so eingerichtet ist, dass sie die aktuellen Fortschritts-Informationen grafisch anzeigt und die aktuelle Zeit, eine verstrichene Zeit, einen verbleibenden Zeitraum sowie einen Überziehungszeitraum grafisch voneinander unterscheidet.

5. Vorrichtung zum Überwachen biologischer Informationen nach Anspruch 4, wobei die CPU des Weiteren so eingerichtet ist, dass sie die aktuellen Fortschritts-Informationen in Form einer analogen Uhr anzeigt.

6. Vorrichtung zum Überwachen biologischer Informationen nach Anspruch 4, wobei die CPU des Weiteren so eingerichtet ist, dass sie die aktuellen Fortschritts-Informationen in Form einer Balkengrafikanzeige anzeigt.

7. Vorrichtung zum Überwachen biologischer Informationen nach Anspruch 1, wobei an dem Eingabeabschnitt ein Eingabe-Kommentar zusammen mit dem Grund und dem Zeitraum eingegeben wird.

8. Vorrichtung zum Überwachen biologischer Informationen nach Anspruch 1, wobei es sich um eine Überwachungsvorrichtung handelt, an der biologische Informationen einer Vielzahl von Patienten auf einem gemeinsamen Anzeigeabschnitt angezeigt und fernüberwacht werden.

## Revendications

1. Moniteur d'informations biologiques adapté à recevoir directement ou par l'intermédiaire d'un réseau des informations biologiques relatives à un patient, qui sont mesurées par une unité fixée au patient, le moniteur d'informations biologiques comprenant :
une partie d'affichage sur laquelle sont affichées les informations biologiques ;
une partie d'entrée pour entrer la raison de l'abandon temporaire d'une pièce, et la période d'abandon temporaire de la pièce, et pour démarrer la période d'abandon temporaire de la pièce ; et
un CPU configuré pour afficher simultanément sur la partie d'affichage la raison de l'abandon temporaire de la pièce accompagnée de l'arrêt de la mesure des informations biologiques du patient, la période d'abandon temporaire de la pièce et, compte tenu de l'heure présente, l'heure prévue pour retourner au lit pour recommencer la mesure, et les informations horaires d'avancement actuel incluant des informations de dépassement d'heure associées à l'heure programmée.

2. Moniteur d'informations biologiques selon la revendication 1, dans lequel
la partie d'entrée comporte une pluralité d'éléments de raison et une pluralité d'éléments de période, et
la raison est choisie parmi les éléments de raison et la période est choisie par les éléments de période.

3. Moniteur d'informations biologiques selon la revendication 1, comprenant en outre : une partie de sortie d'alarme configurée pour fournir en sortie une alarme incluant une alarme visuelle ou audible, dans lequel
dans le cas où la mesure est arrêtée après le temps programmé pour recommencer la mesure, l'alarme est délivrée en sortie.

4. Moniteur d'informations biologiques selon la revendication 1, dans lequel le CPU est configuré en outre pour afficher graphiquement les informations d'avancement actuel et pour distinguer graphiquement les uns des autres l'heure actuelle, le temps écoulé, une période de temps restante et une période de prolongation.

5. Moniteur d'informations biologiques selon la revendication 4, dans lequel le CPU est configuré en outre pour afficher les informations d'avancement actuel sous forme d'une horloge analogique.

6. Moniteur d'informations biologiques selon la revendication 4, dans lequel le CPU est configuré en outre pour afficher les informations d'avancement actuel sous forme d'un affichage par barres graphiques.

7. Moniteur d'informations biologiques selon la revendication 1, dans lequel, dans la partie d'entrée, un commentaire d'entrée est appliqué en entrée en même temps que la raison et la période.

8. Moniteur d'informations biologiques selon la revendication 1, qui est un moniteur dans lequel les informations biologiques d'une pluralité de patients sont affichées sur une partie d'affichage commune et surveillées à distance.
